# EUROPEAN PATENT APPLICATION

(11) **EP 4 502 144 A1**
(43) Date of publication of application: **05.02.2025**
(21) Application number: 23781350.6
(22) Date of filing: 29.03.2023
(51) Int. Cl.: C12N 5/071, C12N 1/04, A01N 1/02, A61K 35/12

(54) **FROZEN AND FREEZE-DRIED MITOCHONDRIA AND USE THEREOF**

(30) Priority: 29.03.2022 KR 20220038921
(71) Applicant: Paean Biotechnology Inc., Daejeon 34013 (KR)
(72) Inventor: KANG, Young Cheol, Wonju-si Gangwon-do 26463 (KR); HAN, Kyuboem, Daejeon 34119 (KR); KIM, Chun-Hyung, Sejong 30150 (KR); KIM, Soomin, Seoul 02754 (KR)
(74) Representative: HGF
(86) International application number: PCT/KR2023/004181
(87) International publication number: WO 2023/191495

(57) **Abstract**

The present invention relates to a method for freezing and/or freeze-drying mitochondria isolated from cells, and a use of frozen and/or freeze-dried mitochondria. The frozen mitochondria according to the present invention showed similarity to non-frozen mitochondria in terms of anti-inflammatory activity and activity of energy metabolism-related enzymes. Also, the freeze-dried mitochondria showed similar levels of anti-inflammatory activity, activity of energy metabolism-related enzymes, and long-term preservation efficacy after recovery to those in the mitochondria before freeze-drying. Therefore, allowing isolated mitochondria to find a wide spectrum of applications in the treatment of various diseases caused by mitochondrial dysfunction or impairment, the frozen and/or freeze-dried mitochondria according to the present invention is expected to greatly enhance the commercial availability of therapeutics containing mitochondria.

## Description

### Technical Field

The present invention relates to a method for freezing and/or freeze-drying mitochondria isolated from cells, and a use of frozen and/or freeze-dried mitochondria. More specifically, the present invention relates to a method for freezing and/or freeze-drying mitochondria, and a pharmaceutical composition and a long-term preservation solution composition, comprising mitochondria frozen and/or freeze-dried by the method as an active ingredient.

### Background Art

Freeze-drying is a drying method in which a sample containing water is frozen and placed under reduced pressure to sublimate and remove the water in the sample, and is used to preserve water-containing substances such as cells or tissues of plants or animals or foodstuffs for a long period of time.

However, when a substance containing water is frozen, water molecules form ice crystals during freezing, and freeze-concentration occurs in which the medium of solutes or impurities in the substance containing water is unevenly diffused, so a cryoprotectant is used to increase the stability of the tissue. However, in the case of eukaryotic cells, even if a cryoprotectant is used, it is difficult to stably preserve cells without damaging the cell structure such as the cell membrane during freeze-drying, and thus there is a problem that the function of the effective components within the cells cannot be maintained.

Mitochondria play a key role in various physiological processes such as synthesis of ATP as an energy source, production of reactive oxygen species, and apoptosis. Therefore, damage to mitochondria may cause various diseases, and most mitochondrial disorders are caused by hereditary or acquired mutations in mitochondrial DNA. For example, mitochondrial function may be altered by swelling due to abnormal mitochondrial membrane potential, oxidative stress due to reactive oxygen species or free radicals, and defects in the oxidative phosphorylation function for energy production in mitochondria, etc. These mitochondrial dysfunctions have been reported to cause mitochondrial genetic disease, inflammatory disease such as rheumatoid arthritis, ischemic disease, infectious disease, heart disease, muscle disease, degenerative disease such as Parkinson's disease and Alzheimer's disease, and the occurrence and metastasis of various cancers.

In order to treat diseases related to these mitochondrial dysfunctions, attempts to isolate mitochondria from cells or tissues and reinject them into the body for therapeutic purposes have been increasing recently (Korean Patent No. 10-2126199, Korean Patent No. 10-2019277).

Although various studies are being conducted to suggest the possibility of treating specific diseases using mitochondria, the development of methods to stably maintain and store mitochondria and application technologies related to stable formulations is insufficient. Therefore, it is necessary to develop a new method that can stably maintain and store mitochondria and bioactive factors contained therein.

### Detailed Description of Invention

### Technical Problem

Accordingly, the present inventors studied a method for efficiently preserving mitochondria for transplantation therapy as part of the treatment of mitochondria-related diseases. As a result, the present inventors have found that mitochondria freeze-dried by the method of the present invention may be stored at room temperature and low temperature, and that the frozen and/or freeze-dried mitochondria maintain the activity of the mitochondria before freezing. Based on the above, the present inventors completed the present invention.

### Solution to Problem

In order to solve the above problem, in one aspect of the present invention, there are provided frozen or freeze-dried mitochondria, and a pharmaceutical composition and a long-term preservation solution composition kit comprising the same.

In another aspect of the present invention, there are provided a method for stably freezing or freeze-drying isolated mitochondria and a cryoprotectant; and a freezing composition comprising the cryoprotectant and mitochondria.

In another aspect of the present invention, there is provided a use of isolated frozen or freeze-dried mitochondria for the prevention or treatment of a mitochondria-related disease.

In another aspect of the present invention, there is provided a method for preventing or treating a mitochondria-related disease, comprising administering isolated frozen or freeze-dried mitochondria.

### Effects of Invention

The frozen and/or freeze-dried mitochondria according to the present invention was confirmed to show similarity to non-frozen mitochondria. In addition, the frozen and/or freeze-dried mitochondria showed similarity to non-frozen mitochondria in terms of anti-inflammatory activity and activity of energy metabolism-related enzymes. In addition, the freeze-dried mitochondria showed similar levels of long-term preservation efficacy to the non-freeze-dried mitochondria. Therefore, allowing isolated mitochondria to find a wide spectrum of applications in the treatment of various diseases caused by mitochondrial dysfunction or impairment, the frozen and/or freeze-dried mitochondria according to the present invention is expected to greatly enhance the commercial availability of therapeutic agents comprising mitochondria.

### Brief Description of Drawings

Figure 1 is a graph showing the results of confirming the anti-inflammatory efficacy of platelet-derived frozen mitochondria.
Figure 2 is a graph showing the results of confirming the anti-inflammatory efficacy of platelet-derived mitochondria isolated from umbilical cord-derived mesenchymal stem cells or human blood, depending on whether they were frozen.
Figure 3 is a graph showing the results of measuring the protein concentration of mitochondria before freeze-drying and the protein concentration of mitochondria whose volume was restored after freeze-drying.
Figure 4 is a drawing showing the results of confirming the location of mitochondria after treating cultured cells with mitochondria isolated from HEK293 cells and freeze-dried.
Figure 5 is a graph showing the results of measuring the activity of mitochondrial energy synthesis-related enzymes according to whether mitochondria isolated from platelets were freeze-dried.
Figure 6 is a graph showing the results of confirming the anti-inflammatory efficacy according to whether mitochondria isolated from platelets were freeze-dried.
Figure 7 is a graph showing the results of confirming the anti-inflammatory efficacy of mitochondria isolated from platelets or umbilical cord-derived mesenchymal stem cells according to whether they were freeze-dried, by concentration.
Figure 8 is a drawing (top) and graph (bottom) showing the results of comparing the protection efficacy of the heart according to whether mitochondria contained in a long-term preservation solution were freeze-dried.
Figure 9 is a drawing showing the results of confirming the concentration of cytochrome C in the supernatant of each mixed preservation solution by Western blot after freezing and thawing mitochondria isolated from umbilical cord-derived mesenchymal stem cells, which were mixed with a TTG mixed preservation solution or a SHE mixed preservation solution.
Figure 10 is a graph showing the results of measuring the activity of each mitochondrial energy synthesis-related enzyme after freezing and thawing mitochondria isolated from umbilical cord-derived mesenchymal stem cells, which were mixed with a TT mixed preservation solution, a TTG mixed preservation solution, a TT mixed preservation solution containing DMSO (TT+DMSO), a TTG mixed preservation solution containing DMSO (TTG+DMSO), or a SHE mixed preservation solution, respectively. *p<0.05, **p<0.01, ***p<0.001 vs CTR, #p<0.05, ##p<0.05, ###p<0.001 vs TTG
Figure 11 is a graph showing the results of confirming the anti-inflammatory efficacy of each mitochondria after freezing and thawing mitochondria isolated from umbilical cord-derived mesenchymal stem cells, which were mixed with a TTG mixed preservation solution or a SHE mixed preservation solution. *p<0.05, **p<0.01, ***p<0.001 vs CTR, #p<0.05, ##p<0.05, ###p<0.001 vs TTG
Figure 12 is a drawing showing the results of confirming the platelet aggregation inhibitory effect of each mitochondria after freezing and thawing mitochondria isolated from umbilical cord-derived mesenchymal stem cells, which were mixed with a TTG mixed preservation solution or a SHE mixed preservation solution.
Figure 13 is a drawing showing the results of confirming the change in total mitochondrial protein amount, ATP synthesis rate, staining rate of mitochondria-specific staining reagent, and expression level of Tom20 after freezing and thawing mitochondria isolated from umbilical cord-derived mesenchymal stem cells, which were mixed with a TTG mixed preservation solution, one month later.

### Best Mode for Carrying out the Invention

### Frozen and/or freeze-dried mitochondria

In one aspect of the present invention, there is provided frozen and/or freeze-dried (lyophilized) mitochondria.

As used herein, the term "mitochondria" are cellular organelles of eukaryotic cells involved in the synthesis and regulation of adenosine triphosphate (ATP), an intracellular energy source. Mitochondria are associated with various metabolic pathways *in vivo,* for example, cell signaling, cell differentiation, cell death, as well as control of cell cycle and cell growth. Therefore, it has been reported that mitochondrial dysfunction or impairment due to genetic, environmental, or unknown causes is related to the development of various diseases such as mitochondria related genetic disease, inflammatory diseases such as rheumatoid arthritis, ischemic diseases, infectious diseases, heart diseases, muscle disease, degenerative diseases such as Parkinson's disease or Alzheimer's disease, and the occurrence and metastasis of various cancers.

The mitochondria may be obtained from a mammal, or may be obtained from a human. Specifically, the mitochondria may be isolated from a cell or tissue, and may be isolated from a blood cell or platelet. In addition, the mitochondria may be normal mitochondria obtained from a cell with normal mitochondrial biological activity. In this case, the mitochondria may be isolated and used after concentrating and disrupting the tissue or cell, or may be isolated and disrupted from a tissue or cell sample that has been freeze-stored and then thawed. In addition, the mitochondria may be isolated from a sample that has been freeze-stored and then thawed after culturing a cell or tissue *in vitro.* For example, the mitochondria may be obtained from a somatic cell, a germ cell, or a stem cell.

Specifically, the somatic cells may be muscle cells, hepatocytes, nerve cells, fibroblasts, epithelial cells, adipocytes, osteocytes, leukocytes, lymphocytes, platelets, or mucosal cells.

In addition, the stem cells are undifferentiated cells having the ability to differentiate into various types of tissue cells, and may be any one selected from the group consisting of mesenchymal stem cells, adult stem cells, dedifferentiated stem cells, embryonic stem cells, bone marrow stem cells, neural stem cells, limbal stem cells, and tissue-derived stem cells, but are not limited thereto. In this case, the mesenchymal stem cells may be obtained from any one selected from the group consisting of umbilical cord, umbilical cord blood, bone marrow, fat, muscle, nerve, skin, synovial fluid, testis, amniotic membrane, and placenta.

In addition, the mitochondria may be obtained from an autologous, allogenic, or xenogenic subject. Specifically, the autologous mitochondria refer to mitochondria obtained from tissues or cells of the same subject. In addition, the allogenic mitochondria refer to mitochondria obtained from a subject that belongs to the same species as the subject and has different genotypes for alleles. In addition, the xenogenic mitochondria refer to mitochondria obtained from a subject that belongs to the different species from the subject. In addition, the xenogenic mitochondria refer to mitochondria obtained from a subject that belongs to the different species from the subject.

In addition, the mitochondria may be mitochondria isolated from cells. In addition, the mitochondria may be intact and have mitochondrial activity.

The frozen mitochondria of the present invention may be thawed in a refrigerator. Specifically, the mitochondria may be thawed at about 0 °C to about 10 °C. In addition, the thawed mitochondria may be stored in a refrigerator. Specifically, the mitochondria may be stored at about 0 °C to about 10 °C.

The freeze-dried mitochondria of the present invention may be stored at room temperature or in a refrigerator. Specifically, the mitochondria may be stored at about 0 °C to about 37 °C. The mitochondria may be stored at about 0 °C to about 8 °C, about 9 °C to about 15 °C, about 16 °C to about 22 °C, about 23 °C to about 29 °C, or about 30 °C to about 37 °C.

In addition, the freeze-dried mitochondria may exhibit mitochondrial activity when recovered and used.

As used herein, the term "recovery" refers to restoring freeze-dried mitochondria to their original state, and may be performed by adding an aqueous solution to the freeze-dried mitochondria. In this case, it may be water for injection, physiological saline, phosphate buffer solution, purified water, or deionized water. It may be preferably deionized water.

In this case, the above recovery process may be performed at room temperature or in a refrigerator. Specifically, it may be performed at about 0 °C to 37 °C. The mitochondria may be performed at about 0 °C to about 8 °C, about 9 °C to about 15 °C, about 16 °C to about 22 °C, about 23 °C to about 29 °C, or about 30 °C to about 37 °C. In addition, the above recovery process may be performed for about 1 minute to about 1 hour, about 1 minute to about 50 minutes, about 1 minute to about 40 minutes, about 1 minute to about 30 minutes, about 1 minute to about 20 minutes, about 1 minute to about 10 minutes, or about 1 minute to about 5 minutes. The above recovery process is not particularly limited as long as the freeze-dried mitochondria are recovered and not dried.

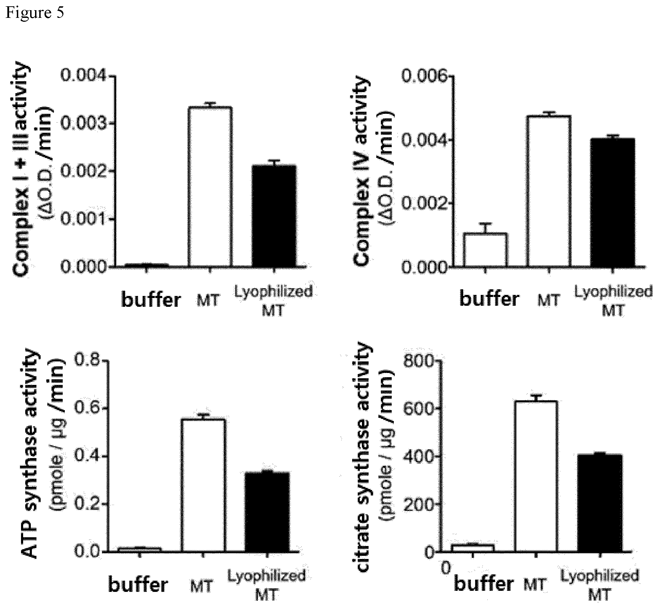

In one embodiment of the present invention, when the freeze-dried mitochondria were recovered and used, it was confirmed that the activity of energy synthesis-related enzymes present in the mitochondria was similar to that of non-freeze-dried mitochondria (Figure 5).

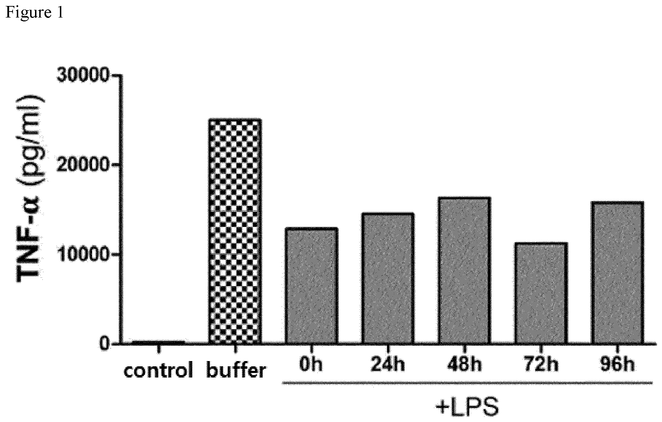

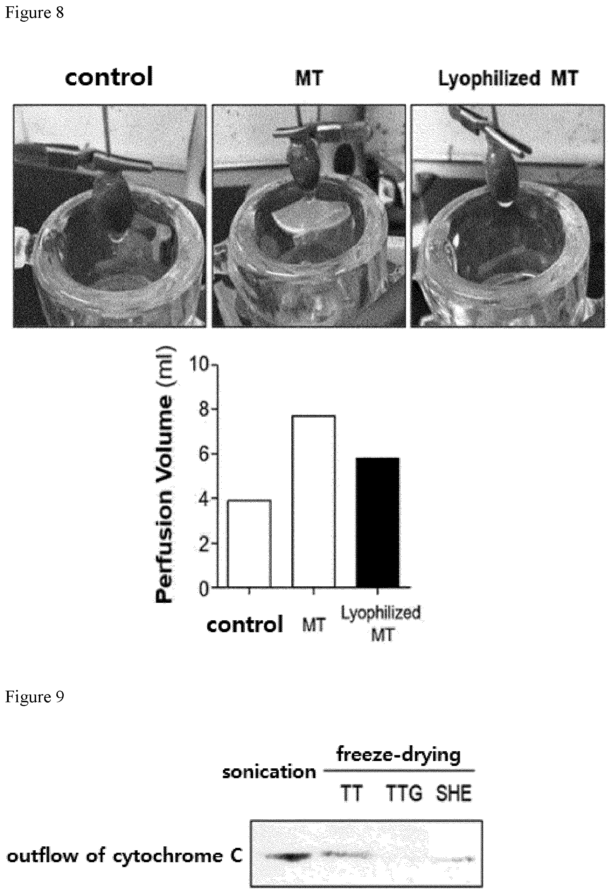

In addition, frozen and/or freeze-dried mitochondria may exhibit anti-inflammatory efficacy and protection efficacy on isolated organs. In one embodiment of the present invention, it was confirmed that when macrophages were treated with frozen and/or freeze-dried mitochondria derived from platelets or umbilical cord-derived mesenchymal stem cells to induce an inflammatory response, the induction of the inflammatory response was inhibited (Figures 1, 2, 6, and 7). In addition, when the isolated heart was stored in a long-term storage solution comprising the freeze-dried mitochondria, it exhibited heart protection efficacy (Figure 8).

### Freezing and freeze-drying method

In another aspect of the present invention, there is provided a method for stably freezing and/or freeze-drying isolated mitochondria.

The freezing method may comprise: isolating mitochondria; and freezing the isolated mitochondria by treating them with a cryoprotectant for mitochondria comprising glycine. In this case, the cryoprotectant may further comprise any one selected from the group consisting of trehalose, tris, and a combination thereof.

In addition, the freeze-drying method may comprise: isolating mitochondria; freezing the isolated mitochondria by treating them with a cryoprotectant for mitochondria comprising glycine; and freeze-drying the frozen mitochondria. Here, the mitochondria are the same as described above. In this case, the cryoprotectant may further comprise any one selected from the group consisting of trehalose, tris, and a combination thereof.

In the case of isolating the mitochondria from a specific cell, the mitochondria may be isolated through a variety of modified methods, including various known methods, for example, using a specific buffer solution or using a electric potential difference and a magnetic field, and the like.

In terms of maintaining mitochondrial activity, the isolation of mitochondria may be performed by disrupting tissues or cells and centrifuging them. In one embodiment, the isolation of mitochondria may be performed by culturing cells and performing a first centrifugation on a composition comprising the cells to produce a pellet, resuspending the pellet in a buffer solution and homogenizing it, performing a second centrifugation on the homogenized solution to produce a supernatant, and performing a third centrifugation on the supernatant to purify the mitochondria. In this case, it is preferable to adjust the time for performing the second centrifugation to be shorter than the time for performing the first and third centrifugations in terms of maintaining cell activity, and the speed may be increased from the first centrifugation to the third centrifugation.

Specifically, the first to third centrifugations may be performed at a temperature of about 0 °C to about 10 °C, preferably at a temperature of about 3 °C to about 5 °C. In addition, the time for performing the centrifugation may be about 1 minute to 50 minutes, and may be appropriately adjusted depending on the number of centrifugations and the content of the sample.

In addition, the first centrifugation may be performed at a speed of about 100 xg to about 1,000 xg, about 200 xg to about 700 xg, or about 300 xg to about 450 xg. In addition, the second centrifugation may be performed at a speed of about 1 xg to about 2,000 xg, about 25 xg to about 1,800 xg, or about 500 xg to about 1,600 xg. In addition, the third centrifugation may be performed at a speed of about 100 xg to about 20,000 xg, about 500 xg to about 18,000 xg, or about 800 xg to about 15,000 xg.

In addition, a pharmaceutically acceptable sugar may be used to stabilize the obtained mitochondria. Specifically, the sugar may be sucrose, mannitol, trehalose, or the like, but is not limited thereto. In addition, as a pH buffering agent, pharmaceutically acceptable tris, HEPES (hydroxyethyl piperazine ethane sulfonic acid), phosphate, and the like may be used, but are not limited thereto.

Meanwhile, additives such as chelators and antioxidants may be used to remove damage caused by ion outflow after obtaining the mitochondria and to suppress oxidative stress, and these may be used without limitation including reagents well known in the art. For example, they may be EDTA, EGTA, citrate, glycine, taurine, ATP, and the like, but are not limited thereto.

In addition, in terms of maintaining mitochondrial activity, the isolation of mitochondria may be performed by thawing and disrupting frozen cells or tissues and centrifuging them. The method for obtaining the mitochondria may be performed by freezing cells or tissues, thawing the cells or tissues, and disrupting the thawed cells and tissues.

Specifically, in the present invention, the isolated mitochondria may be frozen by mixing with a cryoprotectant for mitochondria comprising glycine. In this case, the cryoprotectant may further comprise any one selected from the group consisting of trehalose, tris, and a combination thereof. In the present invention, the cryoprotectant may be used in the form of an aqueous solution, and the aqueous solution may be water for injection, physiological saline, phosphate buffer solution, purified water, or deionized water.

The cryoprotectant may maintain and/or enhance the stability of mitochondria when freezing mitochondria, and may also stably maintain the activity of mitochondria.

The cryoprotectant of the present invention may comprise glycine. The glycine may be included in the cryoprotectant at a concentration of about 15 mM or more, but is not limited thereto. Specifically, it may be included at a concentration of about 15 mM to about 150 mM, at a concentration of about 17 mM to about 130 mM, at a concentration of about 20 mM to about 120 mM, at a concentration of about 22 mM to about 110 mM, or at a concentration of about 25 mM to about 100 mM. In addition, the glycine may be used together with at least one amino acid selected from the group consisting of, but not limited to, histidine, isoleucine, leucine, lysine acetate, methionine, phenylalanine, threonine, tryptophan, valine, alanine, arginine, aspartic acid, cysteine, glutamic acid, proline, serine, and tyrosine.

The cryoprotectant may further comprise a sugar. In this case, the sugar may be at least one selected from the group consisting of sucrose, trehalose, mannitol, sorbitol, glucose, fructose, mannose, maltose, lactose, isomaltose, dextran, and dextrin. Preferably, the sugar may be trehalose. Specifically, trehalose in the cryoprotectant may be included at a concentration of about 40 mM to about 400 mM, at a concentration of about 50 mM to about 350 mM, at a concentration of about 60 mM to about 300 mM, at a concentration of about 70 mM to about 250 mM, or at a concentration of about 80 mM to about 200 mM.

The cryoprotectant may further comprise a buffer. The buffer included in the liquid composition for injection may be selected from the group consisting of a tris buffer, HEPES buffer, a MOPS (3-(N-morpholino)propanesulfonic acid) buffer, and an acetate or phosphate-containing buffer, but is not limited thereto. In this case, the pH of the buffer may be be a range of about 7.0 to about 7.8, a range of about 7.2 to about 7.6, or a range of about 7.3 to about 7.5, but is not limited thereto. Preferably, in the present invention, the buffer may be a tris buffer. Specifically, the cryoprotectant may be included at a concentration of about 5 mM to about 50 mM, at a concentration of about 8 mM to about 40 mM, at a concentration of about 10 mM to about 35 mM, at a concentration of about 13 mM to about 30 mM, or at a concentration of about 15 mM to about 25 mM.

More specifically, the cryoprotectant of the present invention may comprise glycine, and may further comprise any one selected from the group consisting of trehalose, tris, and a combination thereof. In one embodiment, the cryoprotectant may comprise glycine and trehalose. The cryoprotectant may comprise glycine and tris. In one embodiment, the cryoprotectant may comprise glycine, trehalose, and tris. In one embodiment of the present invention, a mixed preservation solution (TTG) comprising glycine, trehalose, and tris reduced damage to mitochondria occurring during the freezing and thawing steps compared to a mixed preservation solution (TT) comprising trehalose and tris (Figures 9 to 12).

In addition, the cryoprotectant may further comprise a chelating agent.

The chelating agent may be at least one selected from the group consisting of injectable grades of EGTA (ethylene glycol tetraacetic acid), EDTA (ethylenediamine tetraacetic acid) and BAPTA (1,2-bis(o-aminophenoxy)ethane-N,N,N',N'-tetraacetic acid), but is not limited thereto. The chelating agent may inhibit damage caused by ion outflow after obtaining mitochondria included in the liquid composition for injection.

In addition, the cryoprotectant may comprise an antioxidant, ATP, magnesium, and the like, which are effective for maintaining the function and activity of mitochondria, as an additive.

The freezing may be performed at a temperature range of, -1 °C or lower, but is not limited thereto. Specifically, it may be performed at a temperature range of about -5 °C to about -200 °C, about -15 °C to about -180 °C, about -25 °C to about -160 °C, about -40 °C to about -140 °C, about -55 °C to about -120 °C, about -60 °C to about -100 °C, or about -70 °C to about -90 °C.

The freezing may be performed using liquid nitrogen (LN₂) or a freezing device. Specifically, it may be performed by rapidly freezing using LN₂ during the refrigeration or freezing process, or by using a freezing device such as a deep freezer or a freezer. The freezing is not particularly limited as long as it is a method capable of refrigerating or freezing mitochondria. When freezing the mitochondria using a freezing device, a freezing container may be used. The freezing container may freeze cells by lowering the temperature by about 1 °C per minute. Preferably, when using a deep freezer, the freezing container may be used. In addition, when freezing the mitochondria using a freezing device, a freezing container may not be used.

The freezing may be performed for about 24 hours or more, about 48 hours or more, or about 96 hours or more, but is not particularly limited as long as the frozen mitochondria have normal activity.

The freeze-drying may be performed by dividing it into freezing the mitochondria and drying the mitochondria, but is not limited thereto. In this case, the freeze-drying may be performed in a vacuum. The freeze-drying of the mitochondria may be performed by freezing the mitochondria in a vacuum and then gradually increasing the temperature.

When freeze-drying the mitochondria, the freezing step may be performed at about-40 °C or lower. Specifically, it may be performed at about -80 °C to about -40 °C, about -70 °C to about -40 °C, about -60 °C to about -40 °C, or about -50 °C to about -40 °C. In this case, the freezing may be performed for about 5 minutes to about 120 minutes, about 10 minutes to about 100 minutes, about 15 minutes to about 80 minutes, about 20 minutes to about 60 minutes, or about 25 minutes to about 40 minutes. In one embodiment of the present invention, the freezing was performed at about -40 °C for 30 minutes.

When freeze-drying the mitochondria, the drying step may be performed in three stages. The first stage drying may be performed at about -30 °C to about 0 °C. More specifically, it may be performed at about -40 °C to about 0 °C, about -30 °C to about -5 °C, or about -20 °C to about -10 °C. In this case, the first stage drying may be performed for about 1 hour to about 50 hours. Specifically, it may be performed for about 1 hour to about 50 hours, about 5 hours to about 50 hours, about 10 hours to about 50 hours, about 20 hours to about 50 hours, or about 30 hours to about 50 hours. In one embodiment of the present invention, the first stage drying was performed at about -20 °C for 25 hours, and was additionally performed at about -20 °C for 8.3 hours depending on the mitochondrial state.

The second stage drying may be performed at about 0 °C to about 10 °C or lower. More specifically, it may be performed at about 0 °C to about 10 °C, about 2 °C to about 9 °C, about 4 °C to about 8 °C, or about 5 °C to about 7 °C. In this case, the second stage drying may be performed for about 1 hour to about 24 hours. Specifically, the second stage drying may be performed for about 1 hour to about 16 hours, about 2 hours to about 16 hours, about 4 hours to about 16 hours, about 6 hours to about 16 hours, or about 8 hours to about 16 hours. In one embodiment of the present invention, the second stage drying was performed at about 6 °C for 8.3 hours.

The third stage drying may be performed at about 11 °C to about 30 °C or lower. More specifically, it may be performed at about 11 °C to about 30 °C, about 15 °C to about 30 °C, about 20 °C to about 30 °C, or about 25 °C to about 30 °C. In this case, the third stage drying may be performed for about 1 hour to about 18 hours. Specifically, the third stage drying may be performed for about 1 hour to about 18 hours, about 3 hours to about 18 hours, or about 6 hours to about 18 hours, or about 9 hours to about 18 hours. In one embodiment of the present invention, the third stage drying was performed at about 25 °C for 9 hours.

The concentration of mitochondria mixed with the cryoprotectant may be from about 0.1 mg to about 10 mg, from about 0.2 mg to about 9 mg, from about 0.3 mg to about 8 mg, from about 0.4 mg to about 7 mg, from about 0.5 mg to about 6 mg, from about 0.6 mg to about 5 mg, from about 0.7 mg to about 4 mg, from about 0.8 mg to about 3 mg, or from about 0.9 mg to about 2 mg per mL.

In addition, the frozen mitochondria may be freeze-dried.

### Cryoprotectant and freezing composition

In another aspect of the present invention, there are provided a cryoprotectant for mitochondria comprising glycine, and a freezing composition comprising the cryoprotectant and mitochondria. In this case, the cryoprotectant may further comprise any one selected from the group consisting of trehalose, tris, and a combination thereof.

The mitochondria and the cryoprotectant are the same as described above.

### Pharmaceutical composition

In another aspect of the present invention, there is provided the pharmaceutical composition for preventing or treating a disease, comprising the frozen and/or freeze-dried mitochondria as an active ingredient.

In this case, a use of the pharmaceutical composition may be for the prevention or treatment of a mitochondria-related disease.

As used herein, the term "mitochondria-related disease" collectively refers to a disease associated with mitochondrial dysfunction or impairment due to genetic, environmental, or unknown causes. For example, the disease may include genetic diseases such as Leber's Hereditary Optic Neuropathy (LHON), Leigh syndrome, Myoclonic Epilepsy Associated with Ragged-Red Fibers (MERRF), Mitochondrial Myopathy, Encephalopathy, Lactacidosis, Stroke (MELAS); degenerative diseases such as Parkinson's disease and Alzheimer's disease, metabolic diseases such as diabetes and obesity, inflammatory diseases such as sepsis, rheumatoid arthritis, osteoarthritis, and systemic inflammatory response syndrome (SIRS), hearing loss, cancer, ischemic disease, neurological disease, heart disease, muscle disease, degenerative disease, fibrosis disease, eye disease, hair loss, nervous system autoimmune disease, and the like, but is not limited thereto.

The cancer may be any one selected from the group consisting of breast cancer, lung cancer, pancreatic cancer, glioblastoma, gastric cancer, liver cancer, colorectal cancer, prostate cancer, ovarian cancer, cervical cancer, thyroid cancer, laryngeal cancer, acute myeloid leukemia, brain tumor, neuroblastoma, retinoblastoma, head and neck cancer, salivary gland cancer, and lymphoma, but is not limited thereto.

In addition, the ischemic disease may include critical limb ischemia, ischemic stroke, ischemic heart disease, ischemic colitis, and the like, but is not limited thereto. In this case, the ischemic disease may be a disease caused by mitochondrial abnormality, and includes all ischemic cell disorders caused by mitochondrial impairment.

The neurological disease may include epilepsy, drug-resistant epilepsy, bipolar disorder or manic depression of bipolar disorder, headache, migraine, anxiety disorder, attention deficit disorder, addictive disease, personality disorder, autism, traumatic brain injury, Friedreich's ataxia, optic atrophy, and the like, but is not limited thereto.

In addition, the infectious disease may include hepatitis B, hepatitis C, human papilloma virus (HPV) infection, cytomegalovirus infection, viral respiratory disease, influenza virus infection, and the like, but is not limited thereto.

In addition, the muscle disease may include MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, muscular hypotonia, muscular weakness, myotonia, and the like, but is not limited thereto. In this case, the muscle disease may include all muscle cell disorders caused by mitochondrial impairment.

The degenerative disease may include Alzheimer's disease, Parkinson's disease, Lou Gehrig's disease (amyotrophic lateral sclerosis), Huntington's disease, multiple sclerosis, brain dysfunction due to immune system disorders, progressive neurodegenerative disease, metabolic brain disease, Niemann-Pick disease, and dementia due to cerebral ischemia and cerebral hemorrhage, but is not limited thereto.

The eye disease may include glaucoma, diabetic retinopathy, age-related macular degeneration, and the like, but is not limited thereto.

The nervous system autoimmune disease may include multiple sclerosis, neuromyelitis optica, acute disseminated encephalomyelitis, ascending myelitis, central myelitis, descending myelitis, transverse myelitis, myasthenia gravis, Guillain-Barre syndrome, autoimmune uveitis, autoimmune encephalopathy, and chronic inflammatory demyelinating polyneuropathy, and the like, but is not limited thereto.

For the pharmaceutical composition comprising the frozen and/or freeze-dried mitochondria of the present invention as an active ingredient, the mitochondria may be included at a concentration of about 0.1 µg/mL to about 500 µg/mL, about 0.2 µg/mL to about 450 µg/mL, or about 0.5 µg/mL to about 400 µg/mL, but is not limited thereto. The inclusion of the mitochondria in the above range may facilitate the dose adjustment of mitochondria upon administration and may further enhance the degree of improvement of the symptoms of a mitochondria-related disease, specifically an inflammatory disease, of a patient. In this case, the dose of mitochondria may be determined through the quantification of mitochondria by quantifying the membrane protein after thawing the frozen mitochondria or recovering the freeze-dried mitochondria. Specifically, the isolated mitochondria may be quantified through the Bradford protein assay [Paper written by James D. McCully ( J Vis Exp. 2014;(91): 51682)].

In particular, the pharmaceutical composition according to the present invention may administer mitochondria in an amount of about 0.01 mg/kg to about 5 mg/kg, about 0.1 mg/kg to about 4 mg/kg, or about 0.25 mg/kg to about 2.5 mg/kg per dose based on the body weight of the subject to be administered, but is not limited thereto. That is, it is most preferable in terms of cell activity that the pharmaceutical composition is administered with the mitochondria in an amount of the above range based on the body weight of the subject in whom a mitochondria-related disease is developed.

In addition, the pharmaceutical composition may be administered 1 to 10 times, 3 to 8 times, or 5 to 6 times, preferably 5 times. In this case, the administration interval may be an interval of 1 to 7 days or 2 to 5 days, preferably an interval of 3 days.

As used herein, the term "prevention" refers to any action that inhibits or delays the onset of cancer by administration of the pharmaceutical composition. In addition, "treatment" refers to any action that ameliorates or beneficially changes cancer symptoms by administration of the pharmaceutical composition.

As used herein, the term "efficacy" may be determined by one or more parameters, such as survival or disease-free survival over a period of time, such as 1 year, 5 years, or 10 years. In addition, the parameter may include the inhibition of the size of at least one tumor in a subject.

The preferred dosage of the pharmaceutical composition varies depending on the condition and body weight of the patient, the severity of disease, the form of drug, the route and duration of administration, but may be appropriately selected by those of ordinary skill in the art. In the pharmaceutical composition for the prevention or treatment of cancer of the present invention, the active ingredient may be included in any amount (effective amount) depending on the use, formulation, purpose of combining, and the like as long as it may exhibit anticancer activity. Here, "effective amount" refers to an amount of an active ingredient capable of inducing an anticancer effect. Such effective amount may be determined experimentally within the ordinary ability of those of ordinary skill in the art.

The pharmaceutical composition of the present invention may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the application (prescription) target may adapt.

When the pharmaceutical composition of the present invention is prepared as a parenteral formulation, it may be formulated in the form of an injection, a transdermal preparation and a nasal inhalant together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used.

The pharmaceutical composition of the present invention may be a preparation for injection. Therefore, the pharmaceutical composition according to the present invention may be manufactured as an injection that is very stable physically or chemically by adjusting the pH using a buffer solution such as an acidic aqueous solution or phosphate, which may be used as an injection, in order to secure product stability according to the distribution of an injection that is prescribed.

Specifically, the pharmaceutical composition of the present invention may comprise water for injection.

The water for injection refers to distilled water prepared for dissolving a solid injection or for diluting a water-soluble injection. It may be glucose injection, xylitol injection, D-mannitol injection, fructose injection, physiological saline, dextran 40 injection, dextran 70 injection, amino acid injection, Ringer's solution, lactic acid-Ringer's solution, or a phosphate buffer solution or sodium dihydrogen phosphate-citrate buffer solution having a pH of about 3.5 to about 7.5.

The pharmaceutical composition of the present invention may further comprise a stabilizer or a solubilizing agent. For example, the stabilizer may be pyrosulfite or ethylenediamine tetraacetic acid, and the solubilizing agent may be hydrochloric acid, acetic acid, potassium phosphate hydroxide, potassium bicarbonate, potassium carbonate, or tris. In one embodiment, the pharmaceutical composition may comprise a mixed preservation solution such as a TTG (trehalose-tris-glycine) solution, which may be commonly used in pharmaceutically acceptable drug preparations.

Specifically, the pharmaceutical composition of the present invention may comprise a liquid composition for injection in addition to frozen and/or freeze-dried mitochondria. In this case, the frozen and/or freeze-dried mitochondria are the same as described above. The pharmaceutical composition of the present invention comprises a liquid composition for injection, thereby forming a composition for preventing or treating a disease related to mitochondrial function, which may suppress thrombosis that may be caused by mitochondrial aggregation, platelet reduction and aggregation, and the like when administering mitochondria through injection, and may maintain and/or enhance the stability of mitochondria, and may also stably maintain the activity of mitochondria.

Here, the liquid composition may comprise glycine. In addition, the liquid composition may further comprise a sugar and/or a buffer.

The glycine may be present in the liquid composition for injection at a concentration of about 15 mM or more, but is not limited thereto, and specifically, may be present at a concentration of about 15 mM to about 150 mM, at a concentration of about 17 mM to about 130 mM, at a concentration of about 20 mM to about 120 mM, at a concentration of about 22 mM to about 110 mM, or at a concentration of about 25 mM to about 100 mM. In addition, the glycine may be used together with at least one amino acid selected from the group consisting of, but not limited to, histidine, isoleucine, leucine, lysine acetate, methionine, phenylalanine, threonine, tryptophan, valine, alanine, arginine, aspartic acid, cysteine, glutamic acid, proline, serine, and tyrosine.

In addition, the sugar included in the liquid composition for injection may be at least one selected from the group consisting of sucrose, trehalose, mannitol, sorbitol, glucose, fructose, mannose, maltose, lactose, isomaltose, dextran, and dextrin, but is not limited thereto. In particular, the sugar may be trehalose, mannitol, or sucrose. Preferably, the sugar may be trehalose.

The buffer included in the liquid composition for injection may be selected from the group consisting of a tris buffer, a HEPES buffer, a MOPS buffer, and an acetate or phosphate-containing buffer, but is not limited thereto. Preferably, the buffer may be an injectable tris buffer.

Preferably, the liquid composition may comprise glycine, trehalose, and tris.

In this case, the pH of the buffer may be in the range of about 7.0 to about 7.8, in the range of about 7.2 to about 7.6, or in the range of about 7.3 to about 7.5, but is not limited thereto.

In addition, the buffer may be present in the liquid composition for injection at a concentration of about 5 mM to about 50 mM, at a concentration of about 8 mM to about 40 mM, at a concentration of about 10 mM to about 35 mM, at a concentration of about 13 mM to about 30 mM, or at a concentration of about 15 mM to about 25 mM, but is not limited thereto.

The above liquid composition for injection may have an osmolarity in the range of about 200 to about 400 mOsm, about 230 to about 380 mOsm, about 250 to about 350 mOsm, about 260 to about 320 mOsm, about 270 to about 330 mOsm, or about 280 to about 300 mOsm. In this case, the osmolarity in the range promotes long-term storage at a temperature of 2 °C to 8 °C or higher, while making the composition suitable for parenteral administration, for example, intravascular, intramuscular, or subcutaneous injection, without causing side effects to a subject.

As used herein, the term "osmolarity" refers to the moles of solute contributing to the osmotic pressure of a solution per kilogram of solvent, and the osmolarity is determined by measuring the freezing point depression of a sample using an osmometer.

In addition, the liquid composition for injection may further comprise a chelating agent.

The chelating agent may be at least one selected from the group consisting of injectable grades of EGTA, EDTA, and BAPTA, but is not limited thereto. The chelating agent may remove damage caused by ion outflow after obtaining mitochondria included in the liquid composition for injection.

In addition, the pharmaceutical composition may comprise an antioxidant, ATP, magnesium, and the like, which are effective for maintaining the function and activity of mitochondria, as an additive.

The pharmaceutical composition of the present invention may be stored in a container selected from the group consisting of a vial, a cartridge, a syringe, and an autoinjector. In addition, the container in which the pharmaceutical composition is stored may be stored at room temperature, at a refrigerated temperature of about 2 °C to about 8 °C, or at about 25 °C to about 40 °C until it is administered to a subject in need of treatment.

The subject may be, but is not limited to, a mammal such as a human, a dog, a cow, a horse, a pig, a sheep, a goat, a cat, a mouse, a rabbit, a rat, and preferably a human.

Meanwhile, the pharmaceutical composition of the present invention is administered in a pharmaceutically effective amount. The term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined according to factors including the health condition of the patient, the type and severity of the disease, the activity of the drug, sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective to treat a disease.

As used herein, the term "administration" refers to introducing a predetermined substance to a subject by an appropriate method, and the route of administration of the composition may be through any general route as long as it may reach a target tissue. It may be intraperitoneal administration, intravenous administration, intramuscular administration, subcutaneous administration, intradermal administration, topical administration, intranasal administration, intrarectal administration, but is not limited thereto.

A preferred dosage of the pharmaceutical composition of the present invention may be administered in an amount of about 0.01 mg/kg to about 5 mg/kg, about 0.1 mg/kg to about 4 mg/kg, or about 0.25 mg/kg to about 2.5 mg/kg of mitochondria per dose based on the body weight of the subject to be administered, but is not limited thereto. That is, it is most preferable in terms of cell activity that the pharmaceutical composition is administered with the frozen and/or freeze-dried mitochondria in an amount in the above range based on the body weight of the subject suffering from a disease. In addition, the pharmaceutical composition may be administered 1 to 10 times, 3 to 8 times, or 5 to 6 times, preferably 5 times. In this case, the administration interval may be an interval of 1 to 7 days or 2 to 5 days, preferably an interval of 3 days. Such dosage should not be construed as limiting the scope of the present invention in any aspect.

In this case, the pharmaceutical composition may be administered parenterally using an 18G to 32G needle in a volume of about 5 ml or less, about 3 ml or less, or about 2 ml or less, but is not limited thereto.

The term "subject" refers to a subject to which the composition of the present invention may be applied (prescribed), and may be a subject suffering from a disease. In addition, the subject may be a mammal such as a rat, a mouse, or a livestock, including a human, but may preferably be a human. In addition to the frozen and/or freeze-dried mitochondria, the composition of the present invention may further comprise any compound or natural extract whose safety has been verified and is known to have a preventive or therapeutic effect on each disease. In this case, the pharmaceutical composition and the compound or natural extract having the activity may be administered simultaneously or sequentially.

In another aspect of the present invention, there is provided a use of the frozen and/or freeze-dried mitochondria for the manufacture of a pharmaceutical composition for preventing or treating a disease. In addition, the present invention may provide a method for preventing or treating a disease, comprising administering the pharmaceutical composition to a subject. Here, the subject may be a mammal, preferably a human. The frozen mitochondria, the freeze-dried mitochondria, the prevention and treatment are the same as described above.

The disease is a mitochondria-related disease, and the specific disease is the same as described above. In this case, the administration may be performed through an injection to the affected area, subcutaneous injection, or intravenous injection. Through this, the pharmaceutical composition according to the present invention may directly supply mitochondria with normal activity to the affected area where the disease has occurred, thereby being useful for increasing the activity of cells with reduced mitochondrial function or regenerating cells with abnormal mitochondrial function, and may be used for the prevention or treatment of a mitochondria-related disease.

### Long-term preservation solution composition kit

In another aspect of the present invention, there is provided a long-term preservation solution composition kit for preservation of isolated cells, tissues or organs, comprising frozen and/or freeze-dried mitochondria and a long-term preservation solution. The frozen and freeze-dried mitochondria are the same as described above.

The long-term preservation solution may comprise at least one selected from the group consisting of histidine, tryptophan, and ketoglutarate. The ketoglutarate may be alpha-ketoglutarate.

The frozen and/or freeze-dried mitochondria of the kit may be mixed with a long-term preservation solution comprising at least one selected from the group consisting of histidine, tryptophan, and ketoglutarate before use.

When the frozen and/or freeze-dried mitochondria of the kit are used in a state mixed with a preservation solution, the concentration of the frozen and/or freeze-dried mitochondria may be from about 0.01 µg/mL to about 1 mg/mL, from about 0.01 µg/mL to about 100 µg/mL, from about 0.01 µg/mL to about 90 µg/mL, from about 0.01 µg/mL to about 80 µg/mL, from about 0.01 µg/mL to about 70 µg/mL, from about 0.01 µg/mL to about 60 µg/mL, from about 0.01 µg/mL to about 50 µg/mL, from about 0.01 µg/mL to about 40 µg/mL, from about 0.01 µg/mL to about 30 µg/mL, from about 0.01 µg/mL to about 20 µg/mL, from about 0.01 µg/mL to about 10 µg/mL, from about 0.01 µg/mL to about 9 µg/mL, from about 0.01 µg/mL to about 8 µg/mL, from about 0.01 µg/mL to about 7 µg/mL, from about 0.01 µg/mL to about 6 µg/mL, from about 0.01 µg/mL to about 5 µg/mL, from about 0.1 µg/mL to about 10 µg/mL, from about 0.5 µg/mL to about 10 µg/mL, from about 1 µg/mL to about 10 µg/mL, from about 2.5 µg/mL to about 10 µg/mL, or from about 5 µg/mL to about 10 µg/mL.

The concentration of histidine in the long-term preservation solution may be from about 1 mM to about 500 mM, from about 5 mM to about 500 mM, from about 10 mM to about 500 mM, from about 25 mM to about 500 mM, from about 50 mM to about 500 mM, from about 75 mM to about 500 mM, from about 100 mM to about 500 mM, from about 125 mM to about 500 mM, from about 150 mM to about 500 mM, from about 175 mM to about 500 mM, from about 198 mM to about 500 mM, from about 100 mM to about 400 mM, from about 100 mM to about 300 mM, from about 100 mM to about 200 mM, or from about 100 mM to about 198 mM.

The concentration of tryptophan in the long-term preservation solution may be from about 1 µM to about 100 mM, from about 1 µM to about 90 mM, from about 1 µM to about 80 mM, from about 1 µM to about 70 mM, from about 1 µM to about 60 mM, from about 1 µM to about 50 mM, from about 1 µM to about 40 mM, from about 1 µM to about 30 mM, from about 1 µM to about 20 mM, from about 1 µM to about 10 mM, from about 1 µM to about 5 mM, from about 10 µM to about 10 mM, from about 100 µM to about 10 mM, from about 0.5 mM to about 10 mM, from about 1 mM to about 10 mM, from about 1.5 mM to about 10 mM, or from about 2 mM to about 10 mM.

The concentration of ketoglutarate in the long-term preservation solution may be from about 1 µM to about 100 mM, from about 1 µM to about 90 mM, from about 1 µM to about 80 mM, from about 1 µM to about 70 mM, from about 1 µM to about 60 mM, from about 1 µM to about 50 mM, from about 1 µM to about 40 mM, from about 1 µM to about 30 mM, from about 1 µM to about 20 mM, from about 1 µM to about 10 mM, from about 1 µM to about 5 mM, from about 10 µM to about 10 mM, from about 100 µM to about 10 mM, from about 250 µM to about 10 mM, from about 500 µM to about 10 mM, from about 750 µM to about 10 mM, from about 1 mM to about 10 mM, from about 1 mM to about 5 mM, or from about 1 mM to about 2 mM.

The cell, tissue, or organ may be derived from a mammal. The mammal may be a human, a cow, a horse, a pig, a dog, a sheep, a goat, or a cat.

The cell, tissue, or organ may be for transplantation into a living body.

The cell may be a muscle cell, a hepatocyte, a fibroblast, an epithelial cell, a nerve cell, an adipocyte, an osteocyte, a leukocyte, a lymphocyte, a stem cell or a mucosal cell.

The tissue may be selected from skin, muscle, cornea, cartilage, and bone.

The organ may be selected from heart, liver, kidney, small intestine, stomach, pancreas, lung, gallbladder, nerve, skin, blood vessel, tooth, eye, and cornea.

The long-term preservation solution composition kit may be for perfusion or reperfusion. The perfusion may be the replacement of protoplasm with an artificial fluid by injecting or continuously flowing a perfusate into isolated cells, tissues, or organs. The reperfusion may be the temporary cessation of blood supply to the isolated cells, tissues, or organs and then the restoration of blood flow.

The long-term preservation solution composition kit may be for parenteral administration. The composition may be a liquid. In addition, the composition may comprise one or more additives. The additives may include excipients, diluents, anti-adherents, preservatives, vehicles, surfactants, or a combination thereof. In addition, the composition may further comprise salts, such as MgCl₂, CaCl₂, NaCl, KCl, or a combination thereof.

The long-term preservation solution composition may comprise an additive that may increase the long-term preservation efficiency of the isolated cells, tissues, or organs. The additive may be water, a sugar (e.g., sucrose, raffinose, starch), a pH buffer (e.g., sodium phosphate, potassium phosphate), ATP, a buffering agent, albumin, or an antioxidant (e.g., vitamin E).

In another aspect of the present invention, there is provided a use of the frozen and/or freeze-dried mitochondria for the manufacture of a long-term preservation solution composition kit. In addition, in another aspect of the present invention, there is provided a method for storing isolated cells, tissues, or organs, comprising: storing the isolated cells, tissues, or organs in the presence of a long-term preservation solution composition comprising the frozen and/or freeze-dried mitochondria.

The mitochondria, the frozen and/or freeze-dried mitochondria, the cell, the tissue, the organ, the long-term preservation solution composition, and the storage are the same as described above.

The storage may be performed in refrigerated conditions. The refrigerated conditions may be from about -4 °C to about 25 °C, from about -4 °C to about 20 °C, from about -4 °C to about 15 °C, from about -4 °C to about 10 °C, from about -2 °C to about 10 °C, from about - 2 °C to about 8 °C, from about 0 °C to about 15 °C, from about 0 °C to about 10 °C, from about 0 °C to about 8 °C, from about 0 °C to about 6 °C, from about 0 °C to about 5 °C, from about 0 °C to about 4 °C, from about 0 °C to about 3 °C, or from about 2 °C to about 5 °C.

The storage may be performed for about 0 hours to about 2 days, about 0 hours to about 42 hours, about 0 hours to about 36 hours, about 0 hours to about 30 hours, about 0 hours to about 24 hours, about 0 hours to about 18 hours, about 0 hours to about 12 hours, about 0 hours to about 9 hours, about 0 hours to about 6 hours, or about 0 hours to about 3 hours.

In this case, when using frozen mitochondria, the method may further comprise: isolating mitochondria; and mixing the isolated mitochondria with a cryoprotectant and freezing them. In addition, when using freeze-dried mitochondria, the method may further comprise: isolating mitochondria; and mixing the isolated mitochondria with a cryoprotectant and freezing them; and a method for freeze-drying the frozen mitochondria. The method for isolating the mitochondria, the method for freezing the isolated mitochondria, and the method for freeze-drying the frozen mitochondria, and the cryoprotectant are the same as described above.

### Mode for Carrying out the Invention

Hereinafter, the present invention will be described in more detail by way of the following examples. However, these examples are intended to exemplify one or more embodiments, and the scope of the present invention is not limited to these examples.

### Preparation Example 1. Preparation of mitochondria

The concentrated platelets, umbilical cord-derived mesenchymal stem cells, or HEK293 cells were physically disrupted, and then precipitated mitochondria were obtained using a differential centrifugation method.

### Example 1. Freezing and freeze-drying of mitochondria

The obtained mitochondria were resuspended in a TTG mixed preservation solution (0.195 M trehalose, 20 mM tris, 65 mM glycine) at a concentration of 1 mg/mL. Thereafter, they were rapidly frozen using LN₂ or frozen at -80 °C. When freezing mitochondria at -80 °C, they were frozen with or without a freezing container (FC). The freeze-drying process was performed using a freeze-dryer in a vacuum while the temperature was sequentially changed to -40 °C, -20 °C, 6 °C, and 25 °C. Specifically, the freezing process was performed at -40 °C for 30 minutes, and then the reaction was performed at -20 °C for 25 hours. While checking the state of the mitochondria, the reaction was performed at -20 °C for an additional 8.3 hours. Next, the reaction was performed at 6 °C for 8.3 hours and at 25 °C for 9 hours, respectively, to finally obtain freeze-dried mitochondria.

### Example 2. Confirmation of anti-inflammatory efficacy of frozen mitochondria

4×10⁵ THP-1 human monocyte cells were cultured in RPMI 1640 medium containing 1% FBS (fetal bovine serum). After 16 to 24 hours of culture, the THP-1 cells were treated with the frozen platelet-derived mitochondria (40 µg) for 24 to 96 hours.

After 1 hour of treatment, an inflammatory cell model was constructed by treating with LPS (lipopolysaccharide) at a concentration of 2 µg/mL. After 4 hours of treatment with LPS, the medium of the THP-1 cells was collected and centrifuged at 20,000 xg for 10 minutes, and the concentration of TNF-α, an inflammatory cytokine, in the supernatant was measured by ELISA (Figure 1). In addition, the anti-inflammatory efficacy of stem cell-derived mitochondria and platelet-derived mitochondria (isolated from the blood of 3 people) was also confirmed using the same method. At this time, the mitochondria were treated at a concentration of 40 µg.

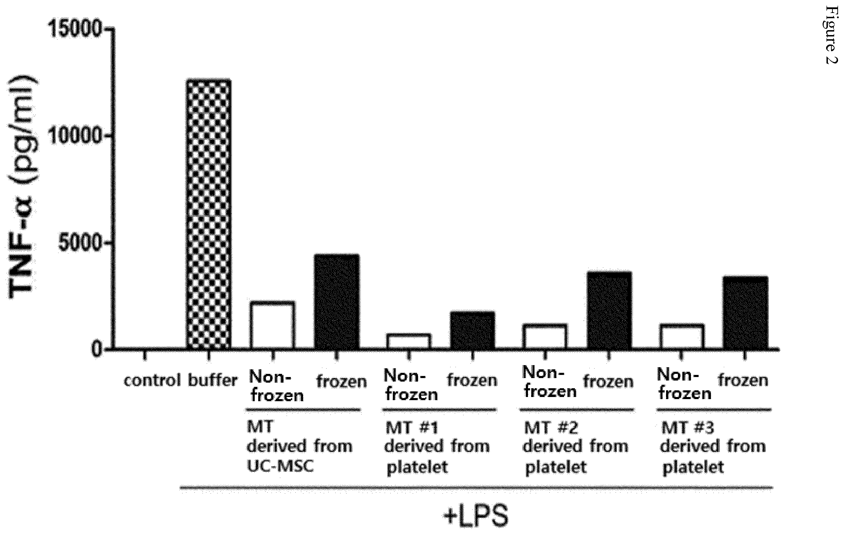

As a result, as shown in Figures 1 and 2, the anti-inflammatory effect of frozen platelet-derived mitochondria or stem cell-derived mitochondria was confirmed compared to the group treated only with the excipient (control).

### Example 3. Recovery of freeze-dried mitochondria

Freeze-dried mitochondria were stored at room temperature and in a refrigerator and were recovered to the volume before freeze-drying by adding ultrapure distilled water just before use.

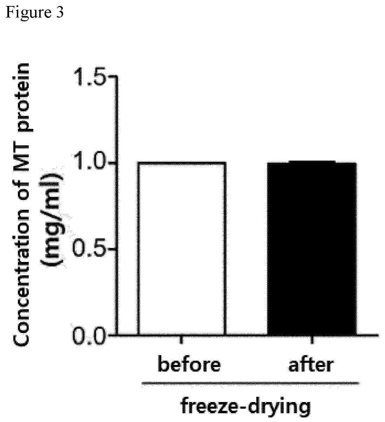

The protein concentration of mitochondria before freeze-drying and the protein concentration of mitochondria after recovery were measured by the BCA (bicinchoninic acid) method (Figure 3).

### Example 4. Confirmation of intracellular movement of freeze-dried mitochondria

Mitochondria were obtained from cells (HEK293-MT^{dsRED}) labeled with red fluorescent protein on the mitochondria, freeze-dried by the method of Example 3, and then recovered. The cultured HEK293-MTS-GFP cells (mitochondria was labeled with green fluorescent labeled protein) were treated with the recovered mitochondria at a concentration of 10 µg for 1 day, and then the cells were observed using a confocal microscope.

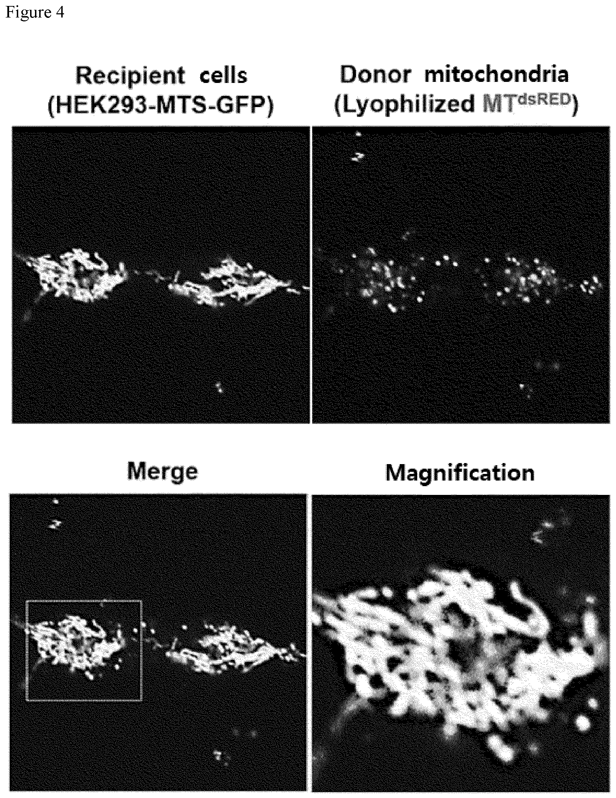

As a result, as shown in Figure 4, the recovered mitochondria after freeze-drying moved into the transplanted cells.

### Example 5. Confirmation of energy synthesis-related enzyme activity of frozen and freeze-dried mitochondria

In order to confirm the change in mitochondrial activity according to freezing or freeze-drying, the protein concentration of freeze-dried or non-freeze-dried platelet-derived mitochondria was measured using the BCA method, and the activity of energy synthesis-related enzymes per the same protein amount was compared.

Specifically, the activity of complex I and complex III, an oxidative phosphorylation complex, present in the mitochondrial inner membrane was evaluated by measuring the intensity of absorbance that changes as oxidized cytochrome c (Sigma aldrich, cat no. C2037) is reduced at a wavelength of 550 nm for 20 minutes at 1-minute intervals. In addition, the activity of complex IV was evaluated by measuring the intensity of absorbance that changes as cytochrome c is reduced using sodium hydrosulfite (Sigma aldrich, cat no. 157953) and then oxidized at a wavelength of 550 nm for 20 minutes at 1-minute intervals. ATP synthesis ability was evaluated by measuring luminescence at 1-minute intervals for 20 minutes using the CellTiter-Glo luminescence kit (Promega) after adding ADP. Mitochondrial citrate synthase activity was measured at 1-minute intervals for 20 minutes, utilizing the principle that acetyl-CoA (Sigma aldrich, cat no. A2181) and oxaloacetic acid (Sigma aldrich, cat no. 04126) are converted to coenzyme A (CoA-SH) through mitochondrial citrate synthase, and that the coenzyme A reacts with DTNB (5,5'-dithiobis(2-nitrobenzoic acid), Sigma aldrich, cat no. D218200) to emit color at a wavelength of 450 nm (Figure 5).

As a result, as shown in Figure 5, it was confirmed that the activity of the enzymes present in the mitochondria was maintained even after freezing or freeze-drying.

### Example 6. Confirmation of anti-inflammatory efficacy of freeze-dried mitochondria

4×10⁵ THP-1 human monocyte cells were cultured in RPMI 1640 medium containing 1% FBS (fetal bovine serum). After 16 to 24 hours of culture, the THP-1 cells were treated with non-freeze-dried mitochondria or freeze-dried mitochondria (40 µg) prepared by the method of Example 3, isolated from platelets or umbilical cord-derived mesenchymal stem cells.

After 1 hour of treatment, an intracellular inflammatory response was induced by treating with LPS at a concentration of 2 µg/mL. After 4 hours of treatment with LPS, the medium of the THP-1 cells was collected and centrifuged at 20,000 xg for 10 minutes, and the concentration of TNF-α, an inflammatory cytokine, in the supernatant was measured by ELISA. In addition, the anti-inflammatory efficacy of freeze-dried or non-freeze-dried mitochondria by concentration was also confirmed using the same method.

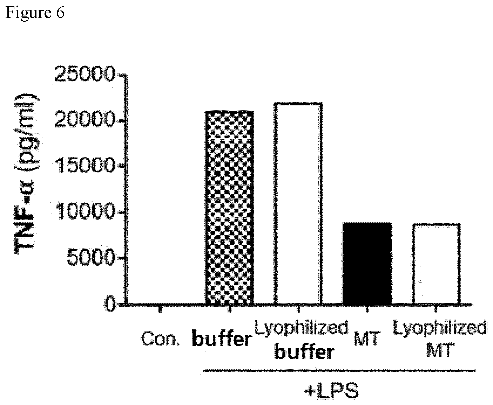

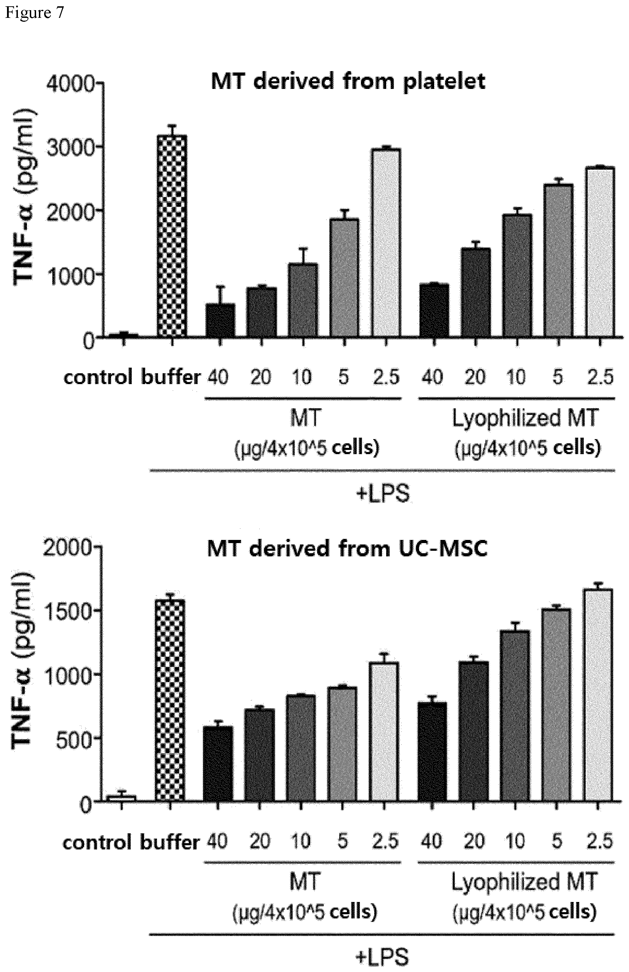

As a result, as shown in Figures 6 and 7, compared to the group treated only with the excipient (control), an anti-inflammatory effect was confirmed in a mitochondrial concentration-dependent manner in the groups treated with non-freeze-dried mitochondria or freeze-dried mitochondria.

### Example 7. Measurement of heart function protection effect of freeze-dried mitochondria

### Example 7.1. Extraction and stabilization of heart

In order to confirm the function of the heart, the heart was first extracted from the rat and stabilized.

Specifically, Sprague-Dawley rats (SD, 12-week-old, male) were anesthetized with ketamine and then sacrificed. The heart of the rat was extracted, and the extracted heart was placed in the Langendorff via an aortic catheter. Thereafter, the extracted heart was washed by perfusion with a Krebs-Henseleit solution for about 20 minutes. The heart was stabilized by injecting a refrigerated stored histidine-tryptophan-ketoglutarate (HTK) solution through the aorta of the heart and perfusion for about 10 minutes. At this time, the HTK solution contained 198 mM histidine, 2 mM tryptophan, 1 mM ketoglutarate, 30 mM mannitol, 4 mM MgCl₂,15 µM CaCl₂, 15 mM NaCl, and 9 mM KCl.

### Example 7.2. Maintenance of perfusate by mitochondria-containing solution in extracted heart

The freeze-dried mitochondria or mitochondria were added to a HTK solution to prepare 5 µg/mL freeze-dried mitochondria-HTK and mitochondria-HTK solutions.

The stabilized hearts prepared in Example 7.1 were injected with a 5 µg/mL mitochondria-HTK solution (MT), a 5 µg/mL freeze-dried mitochondria-HTK solution (freeze-dried MT), or a mitochondria-free HTK solution (control) for about 2 minutes. Thereafter, the hearts were immersed in a 150 µg/30 mL of mitochondria-HTK solution (MT), a freeze-dried mitochondria-HTK solution (freeze-dried MT), or a mitochondria-free HTK solution, respectively, and stored at approximately 4 °C. After 9 hours, the stored hearts were placed in the Langendorff via an aortic catheter, and a Krebs-Henseleit solution was passed through it. The amount of perfusate flowing out of the heart was measured (Figure 8).

As a result, as shown in Figure 8, compared to the case where the hearts were stored only in the HTK solution (control), the amount of the perfusate of the hearts increased by about 1.97 times when stored in the mitochondria-HTK solution and increased by about 1.48 times when stored in the freeze-dried mitochondria-HTK solution.

### Example 8. Confirmation of the effect of reducing mitochondrial damage according to mixed preservation solution during freeze preservation

### Example 8.1. Comparison of mitochondrial protection effects according to mixed preservation solution on freezing and thawing

Mitochondria isolated from umbilical cord-derived mesenchymal stem cells were mixed with a SHE (250 mM sucrose, 20 mM HEPES, 2 mM EGTA) mixed preservation solution, a TTG (20 mM tris, 195 mM trehalose, 66.6 mM glycine) mixed preservation solution, or a TT (20 mM tris, 195 mM trehalose) mixed preservation solution, respectively, frozen, and then thawed, and centrifuged at 12,000 g for 10 minutes. After centrifugation, the supernatant was obtained, and the amount of cytochrome C released when mitochondrial damage was induced was confirmed using Western blot. As a control group, mitochondria disrupted by sonication were centrifuged under the same conditions as above and the supernatant was obtained and used.

As a result, as shown in Figure 9, it was confirmed that the outflow of cytochrome c was increased in the supernatant of mitochondria mixed with a SHE or TT mixed preservation solution and frozen, compared to the supernatant of mitochondria mixed with a TTG mixed preservation solution and frozen and then thawed. Through the above results, it was confirmed that the TTG mixed preservation solution can more stably protect mitochondria from mitochondrial damage during freezing and thawing processes.

### Example 8.2. Comparison of activity of mitochondria frozen and then thawed according to mixed preservation solutions

In order to confirm the mitochondrial protection effect of a TTG mixed preservation solution against stress occurring during freezing and thawing processes, mitochondria isolated from umbilical cord-derived mesenchymal stem cells were mixed with a SHE mixed preservation solution alone, a TTG mixed preservation solution alone, a TT mixed preservation solution alone, a TTG mixed preservation solution containing DMSO (TTG+DMSO), or a TT mixed preservation solution containing DMSO (TT+DMSO), respectively, frozen, and then thawed, and the activity of mitochondria was compared. At this time, DMSO was used at a concentration of 10% as a commonly used freeze preservation solution. Mitochondrial activity was confirmed by measuring the activities of citrate synthase and ATP synthase, which are mitochondria-specific enzymes, in the same manner as in Example 5.

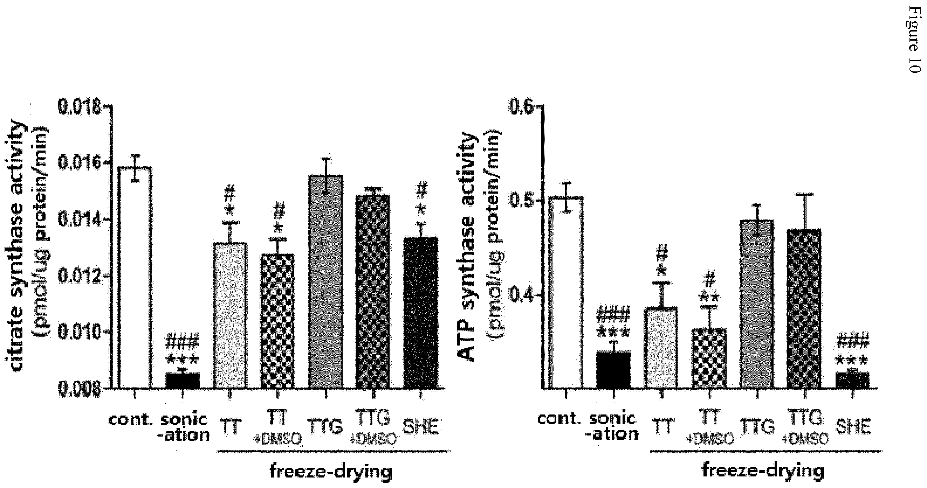

As a result, as shown in Figure 10, the mitochondria mixed in the TTG mixed preservation solution and frozen showed high activities of citrate synthase and ATP synthase compared to the mitochondria mixed in the SHE mixed preservation solution and frozen.

### Example 8.3. Comparison of anti-inflammatory efficacy of mitochondria frozen and then thawed according to mixed preservation solutions

Mitochondria isolated from umbilical cord-derived mesenchymal stem cells were mixed with a SHE mixed preservation solution, a TTG mixed preservation solution alone, a TT mixed preservation solution alone, a TTG mixed preservation solution containing DMSO (TTG+DMSO), or a TT mixed preservation solution containing DMSO (TT+DMSO), respectively, frozen, and then thawed, and the anti-inflammatory efficacy of the mitochondria was confirmed. At this time, the anti-inflammatory efficacy of the mitochondria was confirmed in the same manner as in Example 2.

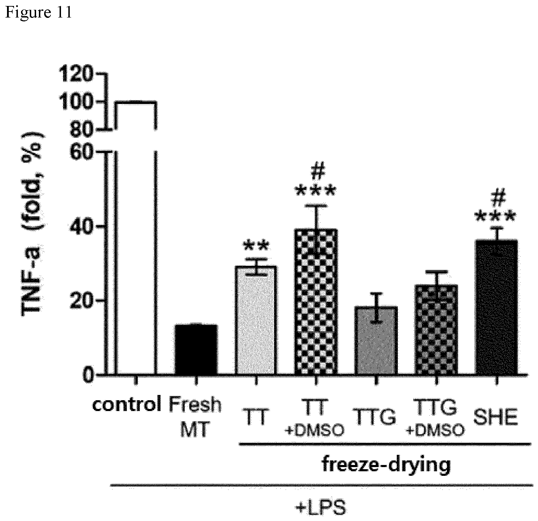

As a result, as shown in Figure 11, the mitochondria mixed in the TTG mixed preservation solution and frozen showed high anti-inflammatory efficacy compared to the mitochondria mixed in the SHE mixed preservation solution, the TT mixed preservation solution, or the TT+DMSO mixed preservation solution and frozen. The above results indicate that the activity of the mitochondria mixed in the TTG mixed preservation solution and frozen is superior to the activity of the mitochondria mixed in other mixed preservation solutions and frozen, suggesting that the TTG mixed preservation solution can protect the mitochondria more stably from the damage to the mitochondria that occurs during the freezing and thawing processes.

### Example 8.4. Comparison of platelet aggregation inhibitory effect of mitochondria frozen and then thawed according to mixed preservation solutions

Mitochondria isolated from umbilical cord-derived mesenchymal stem cells were mixed with a SHE mixed preservation solution or a TTG mixed preservation solution, frozen, and then thawed, and the inhibitory effect of mitochondria on platelet aggregation was compared.

Specifically, the mitochondria thawed after freezing were stained with 0.1 µM MitoTracker Orange for 10 minutes and washed twice with a mitochondrial isolation solution. The mitochondria (5 µg) were mixed with platelets (1 × 10⁷) stained with 1 µM Mitotracker Green and cultured under shaking for one day. The shaken cultured solution (5 µl) was applied to a slide glass and observed using a confocal laser scanning microscope.

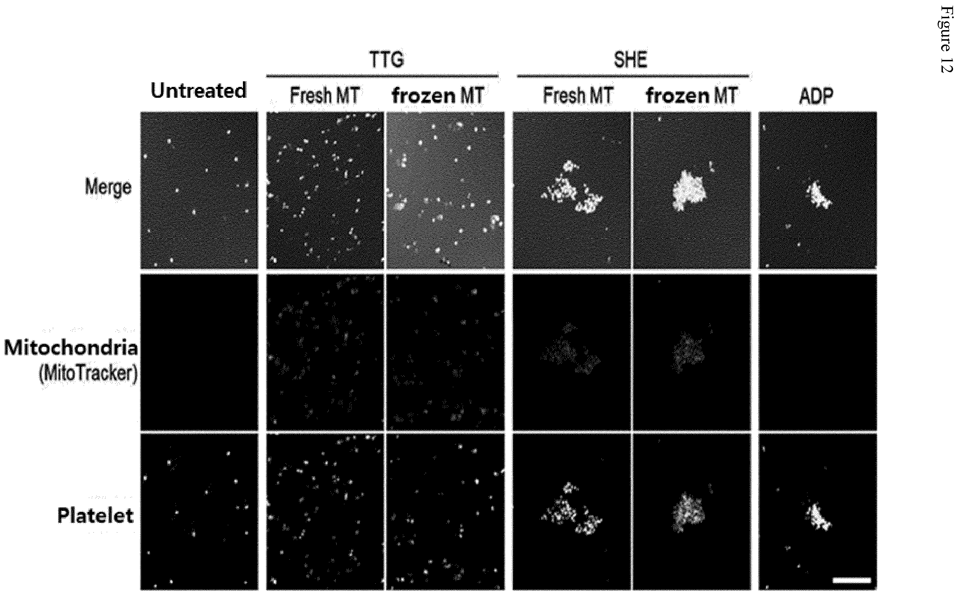

As a result, as shown in Figure 12, in the case of the mitochondria mixed in the TTG mixed preservation solution and frozen, platelet aggregation was not induced. In contrast, in the case of the mitochondria mixed in the SHE mixed preservation solution and frozen, platelet aggregation was induced.

### Example 9. Confirmation of damage to mitochondria mixed with TTG mixed preservation solution, frozen, and thawed

Mitochondria isolated from umbilical cord-derived mesenchymal stem cells were mixed with a TTG mixed preservation solution, frozen, and then thawed one month later, and damage to the mitochondria was confirmed.

Specifically, the total protein amount of mitochondria, ATP synthesis rate, Mitotracker staining rate, and expression level of Tom20 which is a specific protein in the outer membrane were measured, and then the same mitochondria were mixed with a TTG mixed preservation solution and frozen. One month after freezing, the mitochondria were thawed, and the above items were measured again. At this time, the total protein amount of mitochondria was measured using a BCA assay kit, and the ATP synthesis rate was measured using the method of Example 5. The Mitotracker staining rate and Tom20 expression level were analyzed using a flow cytometer after staining with 0.5 µM Mitotracker green or Mitotracker Orange or staining with a Tom20 antibody (Santacruz, sc-17764-FITC) conjugated to a fluorescent protein.

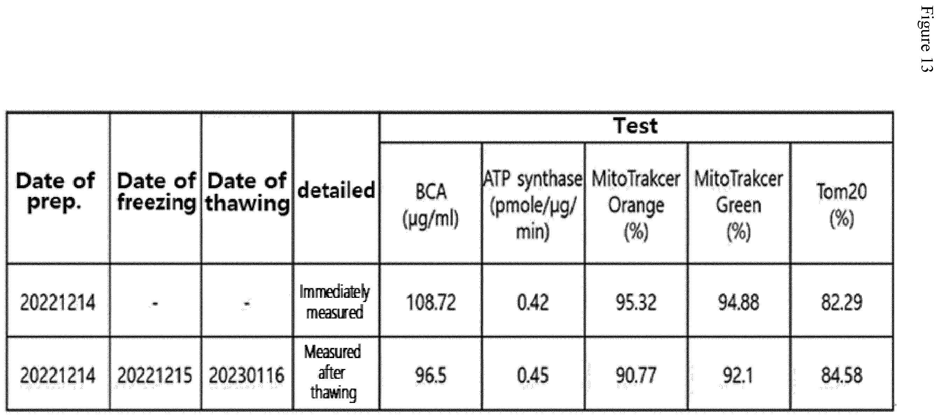

As a result, as shown in Figure 13, it was confirmed that there was no significant change in the total protein amount of mitochondria, ATP synthesis rate, Mitotracker staining rate, and Tom20 expression level before and after mitochondrial freezing when freeze stored in a TTG mixed preservation solution.

## Claims

1. Frozen and/or freeze-dried mitochondria.

2. The frozen and/or freeze-dried mitochondria according to claim 1, wherein the freeze-dried mitochondria are stored at 37 °C or lower.

3. A method for stably freezing isolated mitochondria.

4. The method for freezing mitochondria according to claim 3, wherein the method for freezing mitochondria comprises:
isolating mitochondria; and
freezing the isolated mitochondria by treating them with a cryoprotectant for mitochondria comprising glycine.

5. The method for freezing mitochondria according to claim 4, wherein the cryoprotectant further comprises any one selected from the group consisting of trehalose, tris, and a combination thereof.

6. The method for freezing mitochondria according to claim 4, wherein the freezing is performed at a temperature of -1 °C or lower.

7. The method for freezing mitochondria according to claim 4, wherein the mitochondria are obtained from platelets, muscle cells, hepatocytes, fibroblasts, epithelial cells, nerve cells, adipocytes, osteocytes, leukocytes, lymphocytes, stem cells, or mucosal cells.

8. A method for stably freeze-drying isolated mitochondria.

9. The method for freeze-drying mitochondria according to claim 8, wherein the method for freeze-drying mitochondria comprises:
isolating mitochondria;
freezing the isolated mitochondria by treating them with a cryoprotectant for mitochondria comprising glycine; and
freeze-drying the frozen mitochondria.

10. The method for freeze-drying mitochondria according to claim 9, wherein the cryoprotectant further comprises any one selected from the group consisting of trehalose, tris, and a combination thereof.

11. The method for freeze-drying mitochondria according to claim 9, wherein the freezing is performed at a temperature of -1 °C or lower.

12. The method for freeze-drying mitochondria according to claim 9, wherein the mitochondria are obtained from platelets, muscle cells, hepatocytes, fibroblasts, epithelial cells, nerve cells, adipocytes, osteocytes, leukocytes, lymphocytes, stem cells, or mucosal cells.

13. A cryoprotectant for mitochondria comprising glycine.

14. The cryoprotectant for mitochondria according to claim 13, wherein the cryoprotectant for mitochondria further comprises any one selected from the group consisting of trehalose, tris, and a combination thereof.

15. A composition for freezing mitochondria, comprising a cryoprotectant for mitochondria comprising glycine; and mitochondria.

16. The composition for freezing mitochondria according to claim 15, wherein the cryoprotectant further comprises any one selected from the group consisting of trehalose, tris, and a combination thereof.

17. A pharmaceutical composition for preventing or treating a disease, comprising frozen and/or freeze-dried mitochondria as an active ingredient.

18. The pharmaceutical composition for preventing or treating a disease according to claim 17, wherein the disease is a mitochondria-related disease, including chronic inflammatory disease, acute inflammatory disease, ischemic disease, neurological disease, heart disease, muscle disease, degenerative disease, metabolic disease, fibrosis disease, joint disease, eye disease, hair loss, cancer, hearing loss, and immune-related disease.

19. An long-term preservation solution composition kit for preserving isolated cells, tissues, or organs, comprising frozen and/or freeze-dried mitochondria and a long-term preservation solution.

20. The long-term preservation solution composition kit according to claim 19, wherein the frozen and/or freeze-dried mitochondria are mixed with a long-term preservation solution before use.

21. A use of frozen and/or freeze-dried mitochondria for the prevention or treatment of a mitochondria-related disease.

22. A method for preventing or treating a mitochondria-related disease, comprising administering frozen and/or freeze-dried mitochondria.
